Europäisches Patentamt

European Patent Office

Office européen des brevets

(11) Veröffentlichungsnummer : **0 465 494 B1**

# (12) EUROPÄISCHE PATENTSCHRIFT

(45) Veröffentlichungstag der Patentschrift :
30.12.92 Patentblatt 92/53

(51) Int. Cl.$^5$ : **C12P 7/52,** C12P 7/42

(21) Anmeldenummer : 90904804.3

(22) Anmeldetag : 22.03.90

(86) Internationale Anmeldenummer :
**PCT/EP90/00472**

(87) Internationale Veröffentlichungsnummer :
**WO 90/11362 04.10.90 Gazette 90/23**

---

(54) VERFAHREN ZUR FERMENTATIVEN HERSTELLUNG VON
2-(4-HYDROXIPHENOXI-)PROPIONSÄURE.

---

(30) Priorität : 28.03.89 DE 3910024

(43) Veröffentlichungstag der Anmeldung :
15.01.92 Patentblatt 92/03

(45) Bekanntmachung des Hinweises auf die
Patenterteilung :
30.12.92 Patentblatt 92/53

(84) Benannte Vertragsstaaten :
AT BE CH DE DK ES FR GB IT LI NL

(56) Entgegenhaltungen :
EP-A- 0 133 033
DE-A- 3 532 026
Biochemical Journal, vol. 65, 1957, R.J.W. Byr-de et al "Fungal detoxication. 2. The metabo-lism of somephenoxy-n-alkylcarboxylic acids by aspergillus niger", see page 682, page 686

(73) Patentinhaber : **BASF Aktiengesellschaft**
**Carl-Bosch-Strasse 38**
**W-6700 Ludwigshafen (DE)**

(72) Erfinder : **COOPER, Bryan**
**Waldparkstrasse 32**
**W-6800 Mannhein 1 (DE)**
Erfinder : **LADNER, Wolfgang**
**In den Bellen 5**
**W-6701 Fussgoenheim (DE)**
Erfinder : **HAUER, Bernhard**
**Merowingerstrasse 1**
**W-6701 Fussgoenheim (DE)**
Erfinder : **SIEGEL, Hardo**
**Hans-Purrmann-Allee 25**
**W-6720 Speyer (DE)**

EP 0 465 494 B1

**Beschreibung**

Die Erfindung betrifft ein Verfahren zur Herstellung von 2-(4-Hydroxiphenoxi-)propionsäure

2-(4-Hydroxiphenoxi-)propionsäure ist ein wertvolles Zwischenproduckt, insbesondere für die Herstellung von Herbiziden.

Chemische verfahren zur Herstellung von racemischen, sowie von enantiomeren, reinen Formen von 2-(4-Hydroxiphenoxi-)propionsäure sind bekannt. Diese Verfahren gehen von Hydrochinon aus und besitzen den Nachteil, daß dabei Nebenprodukte entstehen, die mühsam abgetrennt werden müssen. Damit ist eine wirtschaftliche Herstellung nicht möglich.

Es ist bekannt, daß Mikroorganismen aromatische Verbindungen hydroxilieren können. Dabei entstehen in der Regel bishydroxilierte Verbindungen oder Gemische verschiedener Regioisomerer (R.J.W. Byrde, D. Woodcook, Biochem. J. 65, 682 (1957)).

Die regioselektive Oxidation von 2-(Phenoxi-)propionsäure zu 2-(4-Hydroxiphenoxi-)propionsäure durch Mikroorganismen is bisher nicht bekannt.

Überraschenderweise wurde nun gefunden, daß verschiedene Mikroorganismen die preiswerte 2-(Phenoxi-)propionsäure hoch regioselektiv zu 2-(4-Hydroxiphenoxi-)propionsäure oxidieren.

Gegenstand der Erfindung ist ein Verfahren zur fermentativen Herstellung von 2-(4-Hydroxiphenoxi-) propionsäure, das dadurch gekennzeichnet ist, daß man 2-(Phenoxi-)propionsäure oder deren Salze unter aeroben Bedingungen in Gegenwart eines Mikroorganismus oxidiert.

Für das Verfahren eignen sich eine Vielzahl von Mikroorganismen, insbesondere Pilze und Bakterien. Brauchbare Pilze lassen sich beispielsweise aus Erdproben, insbesondere humushaltigen Bodenproben, isolieren. Besonders geeignet für die Umsetzung sind Aspergillus niger-stämme. Weiter eignen sich auch viele Pilze der Gattungen Beauveria, Paecilomyces, Scleroticum und Coprinus, die beispielsweise aus Stammsammlungen bezogen werden können und deren Eignung für die Hydroxylierung sich in einem einfachen Vorversuch ermitteln läßt. Auch geeignete Bakterien lassen sich unter anderem aus Bodenproben isolieren. Als Bakterien kommen insbesondere Streptomyces-Stämme in Betracht.

Zur Durchführung des erfindungsgemäßen Verfahrens werden geeignete Stämme auf ein die 2-(Phenoxi-) propionsäure enthaltendes Nährmedium überimpft und darin bei für den jeweiligen Mikroorganismus günstigen Wachstums- bzw. Produktionsbedingungen aerob inkubiert. Die Fermentation erfolgt kontinuierlich oder diskontinuierlich für 1 bis 10 Tage.

Die Zellen des verwendeten Mikroorganismus, die auch in Form von ruhenden, nicht wachsenden Zellen verwendet werden können, läßt man direkt auf das Substrat einwirken. Es können beliebige, bekannte Inkubationsverfahren angewendet werden, besonders bevorzugt sind aber Fermenter in Form von tiefen, belüfteten und gerührten Tanks. Sehr gute Ergebnisse werden durch Inkubation eines flüssigen Nährmediums erhalten.

Geeignet sind Nährmedien, die Kohlenstoffquellen, Stickstoffquellen, anorganische Salze und gegebenenfalls geringe Mengen an Spurenelementen und Vitaminen enthalten. Als Stickstoffquellen können anorganische bzw. organische Stickstoffverbindungen oder Materialien, die diese Verbindungen enthalten, verwendet werden. Beispiele sind Ammoniumsalze, Nitrate, Maisquellwasser, Bierhefeautolysat, Sojabohnenmehl, Weizengluten, Hefeextract, Hefe, Harnstoff und Kartoffelprotein. Als Kohlenstoffquellen können beispielsweise Zucker wie Glucose, Polyole wie Glycerin oder Fette wie Sojaöl verwendet werden.

Beispiele für anorganische Salze sind die Salze von Calcium, Magnesium, Mangan, Kalium, Zink, Kupfer, Eisen und anderen Metallen. Als Anion der Salze ist besonders das Phosphation zu nennen. Gegebenenfalls werden dem Nährmedium Wachstumsfaktoren zugesetzt, wie z.b. Biotin, Riboflavin oder andere Vitamine.

Das Mischungsverhältnis der genannten Nährstoffe hängt von der Art der Fermentation ab und wird im Einzelfall festgelegt.

Im allgemeinen eignen sich zur Durchführung des erfindungsgemäßen Verfahrens Phenoxipropionsäurekonzentrationen von etwa 1 bis 100 g/l, bevorzugt sind Konzentrationen von etwa 5 bis 50 g/l.

Die Züchtungsbedingungen werden so festgelegt; daß die bestmöglichen Ausbeuten erreicht werden. bevorzugte Züchtungstemperaturen liegen bei 20°C bis 40°C. Besonders vorteilhaft sind Temperaturen zwischen 25°C und 30°C. Vorzugsweise wird der pH-Wert in einem Bereich von 3 bis 9 festgehalten. Besonders vorteilhaft sind pH-Werte zwischen 4 und 7. Im allgemeinen ist eine Inkubationsdauer von 15 bis 100 Stunden ausreichend. Innerhalb dieser Zeit reichert sich die maximale Menge des gewünschten Produkts im Medium an. Die Säure wird vorzugsweise in Form eines Salzes, z.B. des Natriumsalzes, zugegeben. Die Säure kann dem Nährmedium am Anfang auf einmal, nach erfolgtem Wachstum oder während der Züchtung in mehreren Portionen oder kontinuierlich zugegeben werden.

Bevorzugt ist der Einsatz der 2-(Phenoxi-)propionsäure, man kann aber auch deren Salze einsetzen. bevorzugte Salze sind Alkali- und Erdalkalisalze, beispielsweise die Na-, K-, Li-Salze.

Das neue Verfahren eignet sich sowohl zur Oxidation von racemischer 2-(4-Hydroxiphenoxi-)propionsäure als auch von deren Antipoden. Beim erfindungsgemäßen Verfahren bleibt das Assymmetriezentrum unangetastet. Wenn man beispielsweise R-2-(Phenoxi)-propionsäure einsetzt, erhält man unter Erhalt der Konfiguration die entsprechende R-2-(4-Hydroxiphenoxi-)propionsäure. Andere Hydroxylierungsprodukte werden bei der Reaktion nicht beobachtet. Das neue Verfahren stellt somit einen einfachen und kostengünstigeren Weg zur selektiven Herstellung von 2-(4-Hydroxiphenoxi-)propionsäure dar.

Die folgenden Beispiele erläutern die Erfindung:

Beispiel 1

Es wurden zur Überprüfung verschiedener Bakterienstämme zwei flüssige Nährmedien verwendet:

Medium A

20 g/l Glucose
5 g/l Hefeextrakt
5 g/l Ammoniumsulfat
0,5 g/l Magnesiumsulfat-7-hydrat
0,05 g/l Mangansulfat-1-hydrat
1,5 g/l Kaliumdihydrogenphosphat
3,6 g/l Dikaliumhydrogenphosphat
1 g/l (R)-2-(Phenoxi-)propionsäure
2 mg/l Eisen-(II)sulfat-1-hydrat
100 μg/l Zink(II)sulfat-4-hydrat
300 μg/l Borsäure
200 μg/l Cobat(II)chlorid-6-hydrat
10 μg/l Kupfer(II)chlorid-2-hydrat
20 μg/l Nickel(II)chlorid-6-hydrat
30 μg/l Natriummolybdat-2-hydrat

Der pH-Wert wurde mit 5 N Natronlauge auf 6,8 eingestellt. Glucose und Phosphate wurden jeweils getrennt bei 121°C für 10 Minuten autoklaviert. Die (R)-2-(Phenoxi-)propionsäure wurde mit wenig 5 N Natronlauge in Wasser gelöst und sterilfiltriert. Das restliche Medium wurde bei 121°C für 10 Minuten autoklaviert.

Medium B

20 g/l Glucose
40 g/l Maisquellwasser
1,5 g/l Kaliumdihydrogenphosphat
3,6 g/l Dikaliumhydrogensphosphat
1 g/l (R)-2-(Phenoxi-)propionsäure

Der pH-Wert wurde mit 5 N Natronlauge auf 6,8 eingestellt. Glucose und Phosphate wurden jeweils getrennt bei 121°C für 10 Minuten autoklaviert. Die (R)-2-(Phenoxi-)propionsäure wurde mit wenig 5 N Natronlauge in Wasser gelöst und sterilfiltriert. Das restliche Medium wurde bei 121°C für 45 Minuten autoklaviert.

Jeweils 20 ml des sterilen Mediums wurden in sterile 100 ml Erlenmeyerkolben gefüllt, die mit einem sterilen Watteverschluß versehen waren. Jeweils eine Öse der zu prüfenden Bakterienstämme wurde in einen Kolben eingeimpft. Die Kolben wurden dann bei 28°C und 250 UmM für drei bis sieben Tage schüttelnd inkubiert. Anschließend wurden 1000 μl Fermentationsbrühe entnommen und mit 100 μl 1 N HCl und 800 μl Essigsäureethylester versetzt und 15 Sekunden kräftig durchmischt. 700 μl der organischen Phase wurde vorsichtig abgenommen und bei 50°C unter einem sanften Stickstoffstrom im Probegläschen eingedampft. Der Rückstand wurde in 70 μl Essigsäureethylester gelöst und quantitativ in ein Probegläschen für die Gaschromatographie überführt. Hierzu wurden 30 μl N-Methyl-N-trimethylsilyltrifluoracetamid (MSTFA) gegeben. Die Proben wurden dann gaschromatographisch untersucht. Als externer Standard diente eine authentische Probe (R,S)-2-(4-Hydroxiphenoxi-)propionsäure, die chemisch synthetisiert worden war.

Die folgenden Tabelle gibt eine übersicht der Ergebnisse:

| Gattung/Art | | Sammlung/Nummer | Umsatz (%) | Medium |
|---|---|---|---|---|
| Streptomyces | antibioticus | DSM 40725 | 11 | A |
| Streptomyces | antibioticus | ATCC 11891 | 10 | A |
| Streptomyces | flocculus | ATCC 13850 | 18 | A |
| Streptomyces | hygroscopicus | ATCC 19040 | 97 | B |
| Streptomyces | hygroscopicus | ATCC 21705 | 98 | A |
| Streptomyces | kasugaensis | ATCC 15715 | 4 | A |
| Streptomyces | kasugaensis | ATCC 15714 | 23 | B |
| Streptomyces | mediocidicus | ATCC 13279 | 13 | A |
| Streptomyces | niveus | ATCC 19793 | 23 | A |
| Streptomyces | panayensis | ATCC 31055 | 2 | A |
| Streptomyces | roseochromogenes | ATCC 21895 | 4 | A |
| Streptomyces | viridifaciens | ATCC 11989 | 24 | A |

Beispiel 2

Es wurden aus verschiedenen Bodenproben nach der Vorschrift von DREWS (mikrobiologisches Praktikum, 3. Auflage, Springer Verlag, Seite 47-48, 1976) etwa 400 verschiedene, neue Streptomyceten-Stämme isoliert. Diese wurden nach der Vorschrift von Beispiel 1 geprüft. Die folgenden Tabelle gibt eine Übersicht über die Umsätze der als positiv identifizierten Stämme.

| Stammbezeichnung | Umsatz (%) | Medium |
|---|---|---|
| 1034 | 8 | A |
| 1045 | 28 | A |
| 1053 | 22 | A |
| 1057 | 7 | A |
| 1071 | 11 | A |
| 2476 | 13 | A |
| 3279 | 4 | A |
| 3292 | 33 | B |
| 3388 | 26 | B |
| 3520 | 42 | B |
| 3523 | 80 | A |
| 3549 | 80 | B |
| 3558 | 24 | A |
| 3559 | 32 | A |
| 3560 | 98 | A |
| 3561 | 90 | A |
| 3565 | 4 | A |
| 3567 | 72 | B |
| 3574 | 41 | A |
| 3575 | 24 | B |
| 3782 | 44 | A |
| 3925 | 24 | B |
| 4041 | 14 | A |
| 5431 | 19 | A |
| 5432 | 99 | A |
| 5607 | 24 | A |
| 5608 | 26 | A |
| 5613 | 33 | A |
| 5619 | 51 | A |
| 5632 | 18 | A |
| 5635 | 15 | A |
| 5636 | 5 | A |
| 5637 | 39 | A |
| 5638 | 38 | A |
| 5648 | 25 | A |
| 5654 | 59 | A |

Beispiel 3

Es wurde ein flüssiges Nährmedium hergestellt, welches folgende Bestandteile enthielt:
20 g/l Glucose
5 g/l Hefeextrakt
5 g/l Ammoniumsulfat
0,5 g/l Magnesiumsulfat-7-hydrat
0,05 g/l Mangansulfat-1-hydrat
1,5 g/l Kaliumdihydrogenphosphat
3,6 g/l Dikaliumhydrogenphosphat
3 g/l Carbopol® 946 (Carboxyvinylpolymer mit extrem hohem Molekulargewicht)

3 g/l (R)-2-(Phenoxi-)propionsäure
2 mg/l Eisen-(II)sulfat-1-hydrat
100 µg/l Zink(II)sulfat-4-hydrat
300 µg/l Borsäure
200 µg/l Cobat(II)chlorid-6-hydrat
10 µg/l Kupfer(II)chlorid-2-hydrat
20 µg/l Nickel(II)chlorid-6-hydrat
30 µg/l Natriummolybdat-2-hydrat

Der pH-Wert wurde mit 5 N Natronlauge auf 6,8 eingestellt. Glucose und Phosphate wurden jeweils getrennt bei 121°C für 10 Minuten autoklaviert. Die (R)-2-(Phenoxi-)propionsäure wurde mit wenig 5 N Natronlauge in Wasser gelöst und steril filtriert. Das restliche Medium wurde bei 121°C für 10 Minuten autoklaviert. Jeweils 20 ml des sterilen Mediums wurden in sterile 100 ml Erlenmeyerkolben gefüllt, die mit einem sterilen Watteverschluß versehen waren. Eine Öse Sporen des Pilzstammes Aspergillus niger ATCC 11394 wurde in einen Kolben eingeimpft. Der Kolben wurde dann bei 28°C und 250 UpM für drei Tage schüttelnd inkubiert. Anschließend wurden 1000 µl Fermentationsbrühe entnommen und mit 100 µl 1 N HCl und 800 µl Essigsäureethylester versetzt und 15 Sekunden kräftig durchmischt. 700 µl der organischen Phase wurde vorsichtig abgenommen und bei 50°C unter einem sanften Stickstoffstrom im Probegläschen eingedampft. Der Rückstand wurde in 70 µl Essigsäureethylester gelöst und quantitativ in ein Probegläschen für die Gaschromatographie überführt. Hierzu wurden 30 µl N-Methyl-N-trimethylsilyltrifluoracetamid (MSTFA) gegeben. Die Probe wurde dann gaschromatographisch untersucht. Als Referenz diente eine authentische Probe 2-(4-Hydroxiphenoxi-)propionsäure.

Beispiel 4

Es wurde ein flüssiges Nährmedium hergestellt, welches folgende Bestandteile enthielt:
40 g/l Glucose
10 g/l Hefeextrakt
0,5 g/l Magnesiumsulfat-7-hydrat
0,05 g/l Mangansulfat-1-hydrat
1,5 g/l Kaliumdihydrogenphosphat
3,6 g/l Dikaliumhydrogenphosphat
20 g/l (R)-2-(Phenoxi-)propionsäure
2 mg/l Eisen-(II)sulfat-1-hydrat
100 µg/l Zink(II)sulfat-4-hydrat
300 µg/l Borsäure
200 µg/l Cobat(II)chlorid-6-hydrat
10 µg/l Kupfer(II)chlorid-2-hydrat
20 µg/l Nickel(II)chlorid-6-hydrat
30 µg/l Natriummolybdat-2-hydrat

Der pH-Wert wurde mit 5 N Natronlauge auf 6,8 eingestellt. Glucose und Phosphate wurden jeweils getrennt bei 121°C für 10 Minuten autoklaviert. Die (R)-2-(Phenoxi-)propionsäure wurde mit wenig 5 N Natronlauge in Wasser gelöst und steril filtriert. Das restliche Medium wurde bei 121°C für 10 Minuten autoklaviert. 50 ml des sterilen Mediums wurden in einem sterilen 100 ml Erlenmeyerkolben gefüllt, der mit einem sterilen Watteverschluß versehen waren.

Nach Einimpfen von 2,5 ml einer 72 h alten Vorkultur von Beauveria bassiana ATCC 7159 (gezüchtet in gleichem Medium, jedoch in Gegenwart von nur 5 g/l (R)-2-(Phenoxi-)propionsäure, wurde 4-5 Tage bei 28°C und 200 Upm schüttelnd inkuliert. Die sich anschließende gaschromatographische Analyse ergab, daß die eingesetzte Säure zu 98 % hydroxiliert worden war.

Es wurden 98 % der eingesetzten (R)-2-(Phenoxi-)propionsäure zu (R)-2-(4-Hydroxiphenoxi-)propionsäure oxidiert. Bei Einsatz des S-Enantiomeren oder des Racemats der 2-(Phenoxi-)propionsäure wurde dasselbe Ergebnis erzielt.

Beispiel 5

Ein neuer Stamm von Aspergillus niger wurde aus einer humushaltigen Erdprobe nach der Methode von DREWS (Mikrobiologisches Praktikum, 3. Auflage, Springer Verlag, Seiten 51-53 (1976)) isoliert. Dieser Stamm (Nr. 1777) wurde nach der Vorschrift im Beispiel 3 geprüft. Nach drei Tagen waren 99,8 % der eingesetzten Säure oxidiert.

Beispiel 6

Es wurden weitere Stämme der Art Aspergillus niger wie im Beispiel 3 beschrieben geprüft und nach drei bzw. sieben Tagen Fermentationsdauer analysiert. Die folgende Tabelle gibt eine übersicht der Ergebnisse:

| Gattung/Art | Sammlung/Nummer | | Umsatz (%) | Tage |
|---|---|---|---|---|
| Aspergillus niger | ATCC | 9142 | 34 | 3 |
| | ATCC | 11394 | 98 | 3 |
| Aspergillus niger | ATCC | 26693 | 100 | 3 |
| Aspergillus niger | ATCC | 1015 | 83 | 3 |
| Aspergillus niger | ATCC | 10577 | 70 | 3 |
| Aspergillus niger | ATCC | 13794 | 99 | 3 |
| Aspergillus niger | ATCC | 11414 | 79 | 3 |
| Aspergillus niger | ATCC | 26550 | 79 | 3 |
| Aspergillus niger | ATCC | 9029 | 71 | 3 |
| Aspergillus niger | ATCC | 9642 | 41 | 3 |
| Aspergillus niger | ATCC | 32656 | 27 | 7 |
| Aspergillus niger | ATCC | 6275 | 11 | 3 |
| Aspergillus niger | ATCC | 16404 | 97 | 3 |
| Aspergillus niger | ATCC | 10575 | 53 | 7 |
| Aspergillus niger | ATCC | 10581 | 85 | 3 |
| Aspergillus niger | ATCC | 10549 | 27 | 7 |
| Aspergillus niger | ATCC | 1027 | 51 | 7 |
| Aspergillus niger | ATCC | 1040 | 97 | 7 |
| Aspergillus niger | ATCC | 10553 | 14 | 7 |
| Aspergillus niger | ATCC | 10864 | 65 | 7 |
| Aspergillus niger | ATCC | 1004 | 79 | 7 |
| Aspergillus niger | ATCC | 16880 | 99 | 7 |
| Aspergillus niger | ATCC | 16888 | 54 | 7 |
| Aspergillus niger | ATCC | 6273 | 89 | 7 |

| Gattung/Art | Sammlung/Nummer | | Umsatz (%) | Tage |
|---|---|---|---|---|
| Aspergillus niger | ATCC | 7797 | 79 | 7 |
| Aspergillus niger | ATCC | 7983 | 90 | 7 |
| Aspergillus niger | ATCC | 10574 | 100 | 7 |
| Aspergillus niger | ATCC | 10578 | 100 | 7 |

Beispiel 7

Analog dem Beispiel 3 wurden weitere Pilze geprüft und nach drei bzw. sieben Tagen Fermentationsdauer analysiert.

EP 0 465 494 B1

| Gattung/Art | Sammlung/Nummer | | Umsatz (%) | Tage |
|---|---|---|---|---|
| Aspergillus carbonarius | ATCC | 8740 | 7 | 7 |
| Aspergillus carbonarius | ATCC | 6276 | 5 | 7 |
| Aspergillus foetidus | ATCC | 10254 | 62 | 7 |
| Aspergillus parasiticus | ATCC | 26850 | 7 | 3 |
| Aspergillus sclerotiorum | DSM | 63357 | 5 | 3 |
| Aspergillus sclerotiorum | CMI | 112328 | 17 | 3 |
| Aspergillus sclerotiorum | CMI | 116935 | 15 | 3 |
| Beauvarios bassiana | ATCC | 7159 | 99 | 3 |
| Paecilomyces farinosus | ATCC | 26853 | 63 | 7 |
| Sclerotium rolfsii | ATCC | 15206 | 85 | 7 |
| Sclerotium rolfsii | ATCC | 15204 | 19 | 7 |
| Sclerotium rolfsii | ATCC | 15203 | 89 | 7 |
| Sclerotium rolfsii | ATCC | 15201 | 81 | 7 |
| Sclerotium rolfsii | ATCC | 15195 | 85 | 7 |
| Sclerotium rolfsii | ATCC | 26326 | 4 | 7 |
| Sclerotium delphinii | ATCC | 15196 | I | 7 |
| Coprinus cinereus | ATCC | 20120 | 2 | 7 |

## Patentansprüche

1. Verfahren zur fermentativen Herstellung von 2-(4-Hydroxiphenoxi-)propionsäure, dadurch gekennzeichnet, daß man 2-(Phenoxi-)propionsäure oder deren Salze unter aeroben Bedingungen in Gegenwart eines Mikroorganismus oxidiert.

2. Verfahren gemäß Anspruch 1, dadurch gekennzeichnet, daß man als Mikroorganismus einen Pilz verwendet.

3. Verfahren gemäß Anspruch 1, dadurch gekennzeichnet, daß man als Mikroorganismus ein Bakterium verwendet.

4. Verfahren nach einem der Ansprüche 1 bis 3, dadurch gekennzeichnet, daß man die Enantiomeren der 2-(Phenoxi-)propionsäure einsetzt.

## Claims

1. A process for the preparation of 2-(4-hydroxyphenoxy)propionic acid by fermentation, which comprises oxidizing 2-phenoxypropionic acid or salts thereof under aerobic conditions in the presence of a microorganism.

2. A process as claimed in claim 1, wherein a fungus is used as microorganism.

3. A process as claimed in claim 1, wherein a bacterium is used as microorganism.

4. A process as claimed in any of claims 1 to 3, wherein the enantiomers of 2-phenoxypropionic acid are employed.

## Revendications

1. Procédé de préparation fermentative d'acide 2-(4-hydroxyphénoxy-)propionique, caractérisé par le fait

8

que l'on oxyde de l'acide 2-(phénoxy-)propionique ou ses sels dans des conditions aérobies, en présence d'un microorganisme.

2. Procédé selon la revendication 1, caractérisé par le fait que l'on utilise un champignon comme microorganisme.

3. Procédé selon la revendication 1, caractérisé par le fait que l'on utilise une bactérie comme microorganisme.

4. Procédé selon l'une des revendications 1 à 3, caractérisé par le fait que l'on utilise les énantiomères de l'acide 2-(phénoxy)propionique.